# EUROPÄISCHE PATENTANMELDUNG

(11) **EP 4 085 945 A1**
(43) Veröffentlichungstag der Anmeldung: **09.11.2022**
(21) Anmeldenummer: 21172368.9
(22) Anmeldetag: 06.05.2021
(51) Int. Cl.: A61M 5/20, A61M 5/315

(54) **AUTOINJEKTOR MIT VERBESSERTER CO2 BILANZ**

(71) Anmelder: Ypsomed AG, 3401 Burgdorf (CH)
(72) Erfinder: Kaufmann, Nadine, 3400 Burgdorf (CH); Tschirren, Markus, 3400 Burgdorf (CH); Fontanellaz, Thomas, 3303 Zuzwil (CH); Stadelbauer, Bertram, 3454 Sumiswald (CH); Kalirajan, Bravin, 4900 Langenthal (CH)
(74) Vertreter: Meier Obertüfer, Jürg

(57) **Zusammenfassung**

Die Erfindung betrifft einen Autoinjektor zur Ausschüttung eines flüssigen Produkts, insbesondere Medikaments, umfassend:
a. ein Gehäuse (2) und einen in dem Gehäuse (2) angeordneten Produktbehälter (13), insbesondere Spritze, der einen verschiebbaren Kolben (13b) aufweist, wobei der Kolben (13b) in eine Ausschüttrichtung verschiebbar ist, um eine Produktausschüttung des in dem Produktbehälter (13) enthaltenen Produkts zu bewirken,
b. ein Vortriebsglied (7; 7'), das während der Produktausschüttung auf den Kolben (13b) wirkt, und eine erste Feder (9), die auf das Vortriebsglied (7; 7') wirkt,
c. eine Nadelschutzhülse (3), welche für die Auslösung der Produktausschüttung aus einer Ausgangsposition relativ zu dem Gehäuse (2) und entlang einer Längsachse (L) des Autoinjektors in eine proximale Richtung verschiebbar ist, wodurch eine zweite Feder (10) gespannt und insbesondere die Produktausschüttung ausgelöst wird, wobei die Nadelschutzhülse (3) auf die zweite Feder (10) wirkt, wobei
d. das Gehäuse (2) aus einem unter Verwendung einer Mischung aus Recyclingöl und Rapsöl gebildetem Polypropylen oder aus einem unter Verwendung von Tallöl gebildetem Polymerblend enthaltend Polycarbonat und Acrylnitril-Butadien-Styrol gebildet ist.

## Beschreibung

Die Erfindung bezieht sich auf einen Autoinjektor, der oftmals auch als Autoinjektionsvorrichtung bezeichnet wird, mit dem ein in einem Produktbehälter enthaltenes Produkt nach dem Auslösen automatisch ausschüttbar ist, und welcher eine optimierte CO₂-Bilanz bzw. einen optimierten CO₂-footprint aufweist. Bei dem flüssigen Produkt handelt es sich insbesondere um ein Medikament.

### TECHNOLOGISCHER HINTERGRUND

Ein exemplarischer Autoinjektor wie beispielsweise in der internationalen Patentanmeldung PCT/EP2021/052898 beschrieben, weist ein Gehäuse und einen in dem Gehäuse angeordneten Produktbehälter auf. Das Gehäuse kann bei herkömmlichen Ausführungsformen aus einem Polymerblend umfassend Polycarbonat und Acrylnitril-Butadien-Styrol (PC&ABS) gefertigt sein. Bei dem Produktbehälter kann es sich insbesondere um eine Spritze handeln, welche einen Spritzenkörper aufweist, an dessen distalem Ende eine Injektionsnadel fest angeordnet ist.

In bestimmten Ausführungen ist der Produktbehälter entlang der Längsachse unverschiebbar in dem Gehäuse aufgenommen, insbesondere mittels eines Produktbehälterhalters oder Spritzenhalters, der den Produktbehälter axialfest hält und axialfest mit dem Gehäuse verbunden, insbesondere verrastet ist. Der Produktbehälterhalter oder Spritzenhalter kann bei herkömmlichen Ausführungsformen aus einer Polycarbonat / Polyester - Legierung gefertigt sein.

Bevorzugt steht die Nadelspitze über das distale Ende des Gehäuses in distale Richtung über. Hierdurch kann die Nadel mittels einer Bewegung des Gehäuses zum Patienten hin in die Einstichstelle eingestochen werden. Vorzugsweise ist eine Nadelschutzhülse vorgesehen, welche das distale Ende des Autoinjektors bildet und eine Öffnung für die Injektionsnadel aufweist, wobei die Nadel durch die Öffnung hindurchtreten kann. Die Nadelschutzhülse kann bei herkömmlichen Ausführungsformen aus einem Polyoxymethylen (POM) Copolymer gefertigt sein.

Um zu verhindern, dass der Produktbehälter relativ zu dem Produktbehälterhalter oder Spritzenhalter in die proximale Richtung verschiebbar ist, kann ein Mechanikhalter vorgesehen sein, mittels welchem der Produktbehälter gegen den Produktbehälterhalter oder Spritzenhalter gedrückt wird. Der Mechanikhalter kann bei herkömmlichen Ausführungsformen aus einem Polyoxymethylen (POM) Copolymer gefertigt sein.

Ein herkömmlicher Autoinjektor umfasst ferner ein Vortriebsglied, das zumindest während der Produktausschüttung auf den Kolben wirkt, insbesondere an dem Kolben anliegt, und eine erste Feder, die auf das Vortriebsglied wirkt wie z. B. sich insbesondere mit ihrem distalen Ende an dem Vortriebsglied abstützt. Das Vortriebsglied kann bei herkömmlichen Ausführungsformen aus einem Polyoxymethylen (POM) Copolymer gefertigt sein.

Ein herkömmlicher Autoinjektor weist ferner ein Signalglied, einen Signalanschlag und eine zweite Feder auf. Das Signalglied kann bei herkömmlichen Ausführungsformen aus glasfaserverstärktem Polybutylenterephthalat (PBT) gefertigt sein.

Die zweite Feder kann eine auf das Signalglied entgegen der Ausschüttrichtung oder in die proximale Richtung wirkende Federkraft ausüben. Insbesondere kann sich die zweite Feder z. B. mit ihrem proximalen Ende an dem Signalglied abstützen. Die zweite Feder kann sich mit ihrem distalen Ende z. B. an dem Gehäuse oder einem gehäusefesten Element abstützen. Besonders bevorzugt stützt sich die zweite Feder mit ihrem distalen Ende an der Nadelschutzhülse oder einem Element, welches, insbesondere bei Verschiebung der Nadelschutzhülse relativ zu dem Gehäuse, mit der Nadelschutzhülse verschoben wird, ab. Beispielsweise kann das Element ein Schaltmodul oder eine Schalthülse, wie sie weiter unten beschrieben wird, sein. Das Element, insbesondere Schaltmodul, Schalthülse, und/oder eine Sperrhülse, kann bei herkömmlichen Ausführungsformen aus einem Polyoxymethylen (POM) Homopolymer gefertigt sein.

Der Signalanschlag kann von dem Gehäuse oder einem zumindest axialfest mit dem Gehäuse verbundenem Element gebildet werden. Beispielsweise kann dieses Element eine Verschlusskappe am proximalen Ende des Gehäuses sein und/oder das proximale Ende des Autoinjektors bilden. Die Verschlusskappe kann bei herkömmlichen Ausführungsformen aus einem Polymerblend umfassend Polycarbonat und Acrylnitril-Butadien-Styrol (PC&ABS) gefertigt sein.

In bevorzugten Ausführungen kann der Autoinjektor ein Halteelement aufweisen, an dem sich z. B. ein Ende der ersten Feder, insbesondere das proximale Ende der ersten Feder, abstützt. Alternativ kann sich die Feder mit ihrem proximalen Ende an dem Gehäuse oder einem gehäusefesten Element abstützen. Das Halteelement kann bei herkömmlichen Ausführungsformen aus glasfaserverstärktem Polyamid (PA) gefertigt sein.

An dem distalen Ende des Autoinjektors kann in einem Auslieferungszustand eine Abziehkappe angeordnet sein, die vor der Verwendung des Autoinjektors abgezogen, oder abgedreht, und entfernt wird. Die Abziehkappe kann bei herkömmlichen Ausführungsformen aus einem Polymerblend umfassend Polycarbonat (PC) gefertigt sein.

Autoinjektoren wie vorstehend beschrieben sind zur einmaligen Verwendung bestimmt. Aufgrund der Vielzahl kleiner Einzelkomponenten aus unterschiedlichen Materialien, eines Verletzungs- und Infektionsrisikos (insbesondere durch die enthaltene Injektionsnadel) bei einem Zerlegen und weiterer Gründe ist auch ein Komponenten- bzw. Rohstoffrecycling zumindest erschwert. Unter anderem aus den genannten Gründen werden gebrauchte Autoinjektoren häufig einer Verbrennung zugeführt, was gemäss nationalen oder regionalen Gesetzen oder Verordnungen zumindest für institutionelle bzw. kommerzielle Anwender wie z.B. Altersheime oder Krankenhäuser teilweise sogar vorgeschrieben sein kann.

Bei der Verbrennung wird CO₂ freigesetzt, welches bei herkömmlichen Autoinjektoren aus Materialien wie vorstehend beschrieben zuvor in fossilen Rohstoffen gespeichert war, was zu einer ungünstigen CO₂-Bilanz bzw. zu einem ungünstigen CO₂-footprint führt.

Es ist somit eine Aufgabe der Erfindung, einen alternativen Autoinjektor anzugeben, welcher eine verbesserte, insbesondere optimierte, CO₂-Bilanz und einen verbesserten, insbesondere optimierten CO₂-footprint aufweist.

### KURZE BESCHREIBUNG DER ERFINDUNG

Versuche, Autoinjektoren oder zumindest Einzelteile von Autoinjektoren aus Ausgangsmaterialien herzustellen, welche unter Verwendung oder Einsatz von mittels Recyclings oder auf Basis von biologischem Material gewonnener Rohstoffe erzeugt wurden, haben in der Vergangenheit zu wenig überzeugenden Resultaten geführt. Dabei haben sowohl die Ausgangsmaterialien als auch entsprechend hergestellte Autoinjektoren zumindest nicht alle Qualitätsanforderungen erfüllt, welche gemeinhin für eine Produktion von hochwertige Autoinjektoren bzw. an solche Autoinjektoren an sich gestellt werden.

Bezüglich der Ausgangsmaterialien können solche Qualitätsanforderungen insbesondere eine Konstanz und Homogenität von verschiedenen Materialparametern betreffen, welche insbesondere für die Verwendung der Ausgangsmaterialien in einem Spritzgussverfahren relevant sein können, und insbesondere dafür verantwortlich sein können, dass eine Gussform vollständig ausgefüllt bzw. ausgegossen wird, dass ein Aushärten ausreichend schnell und unter hinreichendem Polymerisationsgrad erfolgt, und oder dass ein Schrumpfen oder eine Expansion, welche beim Aushärten erfolgt, sich im Rahmen vertretbarer Toleranzen bewegt.

Bezüglich der Autoinjektoren können solche Qualitätsanforderungen insbesondere eine Oberflächenbeschaffenheit, insbesondere eine (niedrige oder hohe) Oberflächenrauheit, eines oder mehrerer Teile betreffen, welche beispielsweise für eine gute Gleitfähigkeit gegenüber den restlichen und/oder weiteren Teilen benötigt werden kann, aber auch eine sichere Handhabbarkeit gewährleisten soll. Alternativ und oder zusätzlich können Qualitätsanforderungen an die Teile auch eine Steifigkeit, Schlagzähigkeit, Spannungsbeständigkeit und/oder Ähnliches betreffen.

Qualitätsanforderungen können sich schliesslich auch in Maximalwerten für einen akzeptierbaren Ausschuss manifestieren, wobei es sich insbesondere um Prozent- oder Promillewerte handeln kann, welche angeben, wie gross ein maximaler Anteil produzierter Autoinjektoren sein darf, welcher eine (abschliessende) Qualitätskontrolle nicht besteht. Ein solcher Anteil kann hochgradig davon beeinflusst sein, inwieweit Qualitätsanforderungen an Ausgangsmaterialien und/oder Teile erfüllt sind.

Ein häufig im Rahmen einer stichprobenartigen Qualitätskontrolle von Autoinjektoren herangezogener Test ist ein sogenannter kontrollierter Falltest, bei welchem ein intakter (repräsentativer) Autoinjektor mit einer in einer Nadelschutzposition verriegelten Nadelschutzhülse voran aus einer definierten Höhe (insbesondere von 1,0m; 1,1m; 1,2m oder 1,5m) senkrecht auf einen Untergrund mit definierter Härte, insbesondere eine Platte aus Stahl oder einen Boden aus mineralischem Material wie z.B. Stein oder Beton, fallen gelassen wird. Anschliessend wird der Autoinjektor auf Beschädigungen überprüft, insbesondere an einer Schnittstelle zwischen einem Gehäuse und einer Endkappe. Ist die Endkappe verrutscht, insbesondere in einer Richtung von der Nadelschutzhülse weg, oder hat sich gar vollständig vom Gehäuse gelöst, gilt der Falltest als nicht bestanden.

Der kontrollierte Falltest ist insoweit auch von praktischer Relevanz, als ein unbeabsichtigtes (und unkontrolliertes) Fallenlassen von Autoinjektoren beim täglichen Einsatz durchaus auftritt, was häufig dazu führt, dass der Autoinjektor mit der verriegelten Nadelschutzhülse voran auf einem mehr oder weniger harten Untergrund (Stein, Parkett, Laminat oder Teppich) aufschlägt. Ein hochwertiger Autoinjektor soll dabei idealerweise bedenkenlos und ohne irgendwelche Einschränkungen einer Funktionalität und/oder Präzision wie vorbestimmt eingesetzt werden können.

Im Rahmen der Entwicklung von herkömmlichen Autoinjektoren hat sich in umfangreichen Testreihen umfassend kontrollierte Falltests herausgestellt, dass Qualitätsanforderungen insbesondere im Hinblick auf den kontrollierten Falltest am besten mit Materialien erreicht werden können, die einerseits eine möglichst hohe Kerbschlagzähigkeit nach Charpy aufweisen, die insbesondere in einem Bereich oberhalb von 5.5 kJ/m² liegen muss, andererseits eine Steifigkeit in einem Bereich zwischen 2000 und 2400 MPa.

Diesen Erkenntnissen folgend wurden zu Testzwecken Autoinjektoren mit Gehäusen aus verschiedenen Ausgangsmaterialien mit reduzierter CO₂-Bilanz bzw. reduziertem CO₂-footprint hergestellt, und anschliessend ausgiebigen kontrollierten Falltests unterzogen. Dabei wurden jeweils 15 Autoinjektoren zunächst aus einer Höhe von 1,5m auf eine Stahlplatte fallen gelassen. Falls dabei Beschädigungen aufgetreten sind, wurde die Höhe schrittweise reduziert. Andernfalls wurde davon ausgegangen, dass Falltests aus niedrigeren Höhen zu 100% erfolgreich sind.

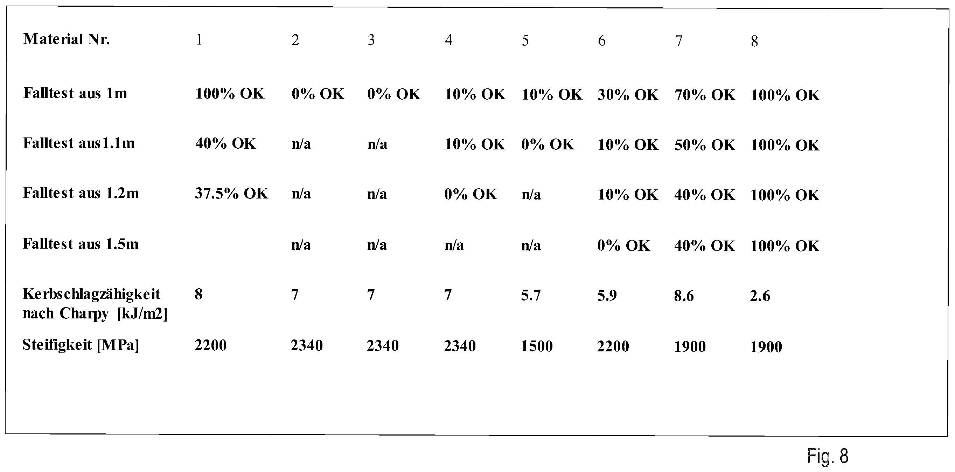

Die Tabelle in Fig.8 zeigt Falltestergebnisse sowie die Kerbschlagzähigkeit nach Charpy in kJ/m² und die Steifigkeit in MPa für verschiedene Gehäuse-Ausgangsmaterialien.

Wie ersichtlich, sind die Falltest-Ergebnisse für Autoinjektoren mit Gehäuse aus den Materialien Nr. 1 bis 4 und 6 unbefriedigend, obwohl sowohl Kerbschlagzähigkeit als auch Steifigkeit klar innerhalb des für herkömmliche Autoinjektoren ermittelten Bereichs liegen. Ebenfalls unbefriedigend sind die Ergebnisse für Material 5, wobei hier die Steifigkeit klar ausserhalb des für herkömmliche Autoinjektoren ermittelten Bereichs liegt.

Auch die Ergebnisse für Material 7 mit einer Steifigkeit unterhalb des für herkömmliche Autoinjektoren ermittelten Bereichs sind unbefriedigend.

Völlig überraschend ergeben sich jedoch sehr positive Testergebnisse für Material 8, obwohl sowohl Kerbschlagzähigkeit nach Charpy als auch Steifigkeit klar ausserhalb der für herkömmliche Autoinjektoren ermittelten Bereiche liegen.

Bei Material 8 handelt es sich um Purell^{™} HP671T renewable, erhältlich von der Firma LyondellBasell^{™}, ein Polypropylen, welches aus Recyclingöl und Rapsöl hergestellt wird.

Ein erfindungsgemässer Autoinjektor zur Ausschüttung eines flüssigen Produkts, insbesondere Medikaments, umfasst demnach:
a. ein Gehäuse und einen in dem Gehäuse angeordneten Produktbehälter, insbesondere Spritze, der einen verschiebbaren Kolben aufweist, wobei der Kolben in eine Ausschüttrichtung verschiebbar ist, um eine Produktausschüttung des in dem Produktbehälter enthaltenen Produkts zu bewirken,
b. ein Vortriebsglied, das während der Produktausschüttung auf den Kolben wirkt, und eine erste Feder, die auf das Vortriebsglied wirkt,
c. eine Nadelschutzhülse, welche für die Auslösung der Produktausschüttung aus einer Ausgangsposition relativ zu dem Gehäuse und entlang einer Längsachse des Autoinjektors in eine proximale Richtung verschiebbar ist, wodurch eine zweite Feder gespannt und insbesondere die Produktausschüttung ausgelöst wird, wobei die Nadelschutzhülse auf die zweite Feder wirkt, wobei
d. das Gehäuse gebildet ist
   i. aus einem unter Verwendung einer Mischung aus Recyclingöl und Rapsöl gebildetem Polypropylen, insbesondere von Purell^{™} HP671T renewable, erhältlich von der Firma LyondellBasell^{™}, oder
   ii. aus einem unter Verwendung von Tallöl gebildetem Polymerblend enthaltend Polycarbonat und Acrylnitril-Butadien-Styrol, insbesondere von Cycoloy^{™} HCX1640 renewable, erhältlich von der Firma Sabic^{™}.

Ein derartiger Autoinjektor erfüllt also Qualitätsanforderungen welche üblicherweise an Autoinjektoren für den einmaligen Gebrauch gestellt werden, in mindestens gleicher Weise wie herkömmliche, insbesondere ausschliesslich unter Verwendung von Ausgangsmaterialien aus fossilen Rohstoffen gebildete Autoinjektoren. Insbesondere haben derartige Autoinjektoren umfassende kontrollierte Falltests wie oben beschrieben zu 100% erfolgreich absolviert.

Ein derartiger Autoinjektor weist zudem eine verbesserte CO₂-Bilanz und/oder einen verbesserten CO₂-footprint auf. Die vorgeschlagenen Ersatzmaterialien sind auch für andere rein mechanische medizinische Einweggeräte geeignet und führen zu einer verbesserten CO₂-Bilanz bei unverminderter Falltestsicherheit, z. B. für Injektoren zur mehrfachen Abgabe einer einstellbaren Dosis aus einem nicht-ersetzbaren Reservoir.

Ein derartiger Autoinjektor kann ferner unter Verwendung von Werkzeugen, insbesondere Spritzgussformen, und/oder Herstellungsprozessen, wie sie bei der Herstellung von unter ausschliesslicher Verwendung von Ausgangsmaterialien aus fossilen Rohstoffen gebildeten Autoinjektoren zum Einsatz kommen,

Bei einem erfindungsgemässen Autoinjektor an einem distalen Ende des Autoinjektors eine Abziehkappe angeordnet sein, welche vor der Verwendung des Autoinjektors abgezogen, abgedreht oder auf andere Weise entfernt werden kann, und welche aus einem unter Verwendung einer Mischung aus Recyclingöl und Rapsöl gebildeten Polypropylen, insbesondere von Purell^{™} HP373P renewable, erhältlich insbesondere von der Firma LyondellBasell^{™}, oder aus einem unter Verwendung von Tallöl gebildeten Polycarbonat, insbesondere von Lexan^{™} HP2REU renewable, erhältlich insbesondere von der Firma Sabic^{™}, gebildet sein kann.

Bei einem erfindungsgemässen Autoinjektor kann die Nadelschutzhülse aus einem unter Verwendung von Biogas gebildeten Polyoxymethylen-Copolymer, insbesondere Hostaform^{™} MT12R01 renewable, erhältlich insbesondere von der Firma Celanese^{™}, gebildet sein.

Bei einem erfindungsgemässen Autoinjektor kann einen Spritzenhalter vorgesehen sein, der den Produktbehälter axialfest hält und axialfest mit dem Gehäuse verbunden ist, und welcher insbesondere aus einer unter Verwendung von Tallöl gebildeten Poycarbonat / Polyester-Legierung, insbesondere Cylex^{™} HX7509HP renewable, erhältlich insbesondere von der Firma Sabic^{™}, gebildet ist.

Ein erfindungsgemässer Autoinjektor kann an einem proximalen Ende des Gehäuses eine Verschlusskappe aufweisen, welche insbesondere form-, kraft- oder stoffschlüssig mit dem Gehäuse verbunden ist, und welche insbesondere aus einem unter Verwendung von Tallöl gebildeten Polymerblend enthaltend Polycarbonat und Acrylnitril-Butadien-Styrol, insbesondere von Cycoloy^{™} HCX1640 renewable, erhältlich insbesondere von der Firma Sabic^{™}, gebildet ist.

Bei einem erfindungsgemässen Autoinjektor kann das Vortriebsglied aus einem unter Verwendung von Biogas gebildeten Polyoxymethylen-Copolymer, insbesondere Hostaform^{™} MT12R01 renewable, erhältlich insbesondere von der Firma Celanese^{™}, gebildet sein.

Bei einem erfindungsgemässen Autoinjektor kann ein Halteelement vorgesehen sein, welches eine Bewegung des Vortriebsglieds relativ zu dem Halteelement und/oder dem Gehäuse in eine Ausschüttrichtung vor einem Auslösen der Produktausschüttung verhindert, wobei das Halteelement insbesondere aus einem glasfaserverstärktem, unter Verwendung von aus fossilen Rohstoffen gebildeten Polyamid, insbesondere von Grilamid^{™} LV-65H FWA, erhältlich insbesondere von der Firma EMS, gebildet ist.

Bei einem erfindungsgemässen Autoinjektor kann nach ein Schaltmodul vorgesehen sein, welches insbesondere aus einem unter Verwendung von Biogas gebildeten Polyoxymethylen-Copolymer, insbesondere Hostaform^{™} MT12R01 renewable, erhältlich insbesondere von der Firma Celanese^{™}, oder aus einem unter Verwendung von Biogas gebildeten Polyoxymethylen-Homopolymer, insbesondere Delrin^{™} SC698 renewable, erhältlich insbesondere von der Firma DuPont^{™}, gebildet ist, und insbesondere eine Sperrhülse und/oder eine Schalthülse umfasst.

Ein erfindungsgemässer Autoinjektor kann ein Signalglied umfassen, welches insbesondere aus einem unter Verwendung von Therephthalat aus PET-Flaschen-Recycling gebildeten, glasfaserverstärkten Polybutylenterephthalat (PBT), insbesondere Elcrin^{™} WF006JiQ, erhältlich insbesondere von der Firma Sabic^{™}, gebildet ist.

Ein erfindungsgemässer Autoinjektor, bei welchem sämtliche vorstehend genannten Elemente bzw. Bauteile aus den genannten Materialien gebildet sind, weist eine optimierte CO₂-Bilanz und/oder einen optimierten CO₂-footprint auf.

Ein erfindungsgemässer Autoinjektor kann insbesondere strukturell, geometrisch und/oder funktional in identischer Weise wie im Abschnitt TECHNOLOGISCHER HINTERGRUND beschrieben ausgeführt sein, wobei ein oder mehrere der im vorliegenden Abschnitt genannten Ausgangsmaterialien zum Einsatz kommen.

Ein erfindungsgemässes Set von Autoinjektoren zur Ausschüttung eines flüssigen Produkts, insbesondere Medikaments, umfasst:
a. einen ersten Autoinjektor wie vorstehen im vorliegenden Abschnitt beschrieben,
b. einen zweiten Autoinjektor wie im Abschnitt TECHNOLOGISCHER HINTERGRUND beschrieben.

Bei einem derartigen Set können Gehäuse, Produktbehälterhalter bzw. Spritzenhalter, Nadelschutzhülse, Mechanikhalter, Vortriebsglied, Signalglied, Schaltmodul, Schalthülse, Sperrhülse, Verschlusskappe, Halteelement, und/oder Abziehkappe des ersten und zweiten Autoinjektors geometrisch, strukturell und/oder funktional paarweise zueinander identisch sein; insbesondere können sämtliche funktional einander entsprechenden mechanischen Bestandteile des ersten und zweiten Autoinjektors geometrisch und/oder strukturell paarweise zueinander identisch sein.

### BESCHREIBUNG DER ZEICHNUNGEN

Die Erfindung und der weitere Aspekt zur Erfindung wurden anhand mehrerer bevorzugter Ausführungen beschrieben. Im Folgenden werden besonders bevorzugte Ausführungen anhand von Figuren beschrieben. Die dabei offenbarten Merkmale bilden den Gegenstand der Erfindung einzeln und in jeglicher Merkmalskombination vorteilhaft weiter. Es zeigen:
- Figur 1a-1b: Explosionsdarstellungen eines Autoinjektors gemäß einer besonders bevorzugten Ausführungsform;
- Figuren 2a-2c: den Autoinjektor aus Figur 1 in einem Auslieferungszustand, wobei die Figuren 2a bis 2c durch die Längsachse der Vorrichtung verlaufende Schnittansichten sind, wobei die Schnittansichten um die Längsachse winkelversetzt sind;
- Figuren 3a-3c: die Vorrichtung und die Ansichten aus den Figuren 2a-2c, wobei sich eine Nadelschutzhülse in ihrer betätigten Position befindet;
- Figuren 4a-4c: die Vorrichtung und die Ansichten aus den Figuren 2a-2c, wobei das Vortriebsglied am Ende eines ersten Teilhubs seines Ausschütthubs gezeigt wird;
- Figuren 5a-5c: die Vorrichtung und die Ansichten aus den Figuren 2a-2c, wobei ein Vortriebsglied am Ende seines Ausschütthubs gezeigt wird;
- Figuren 6a-6c: die Vorrichtung und die Ansichten aus den Figuren 2a-2c, wobei ein Signal, welches das Ende der Produktausschüttung signalisiert, erzeugt wird;
- Figuren 7a-7c: die Vorrichtung und die Ansichten aus den Figuren 2a-2c, wobei die Nadelschutzhülse in ihrer Nadelschutzposition ist, und
- Figur 8: eine Tabelle mit Falltestergebnissen zu unterschiedlichen Gehäusematerialien.

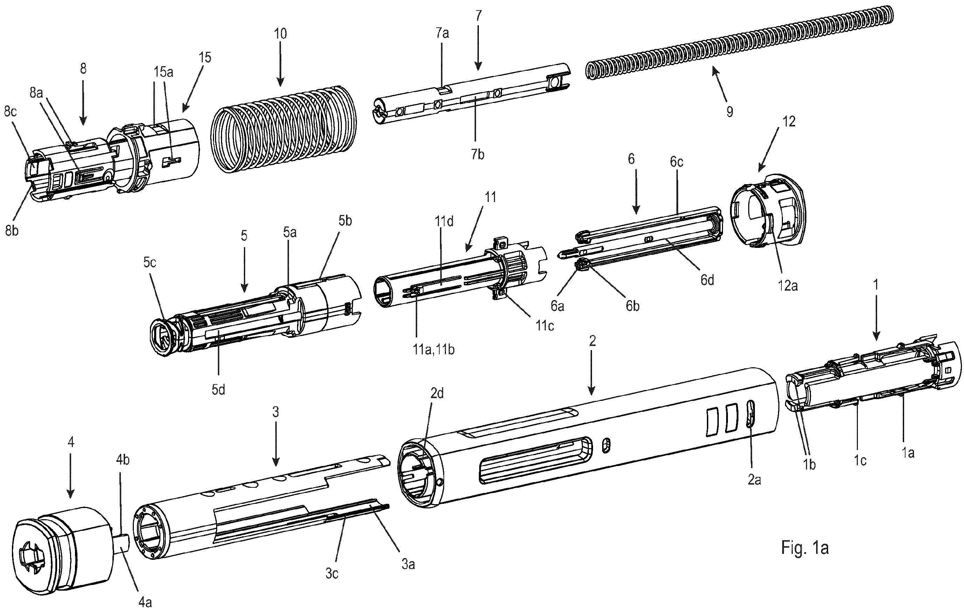

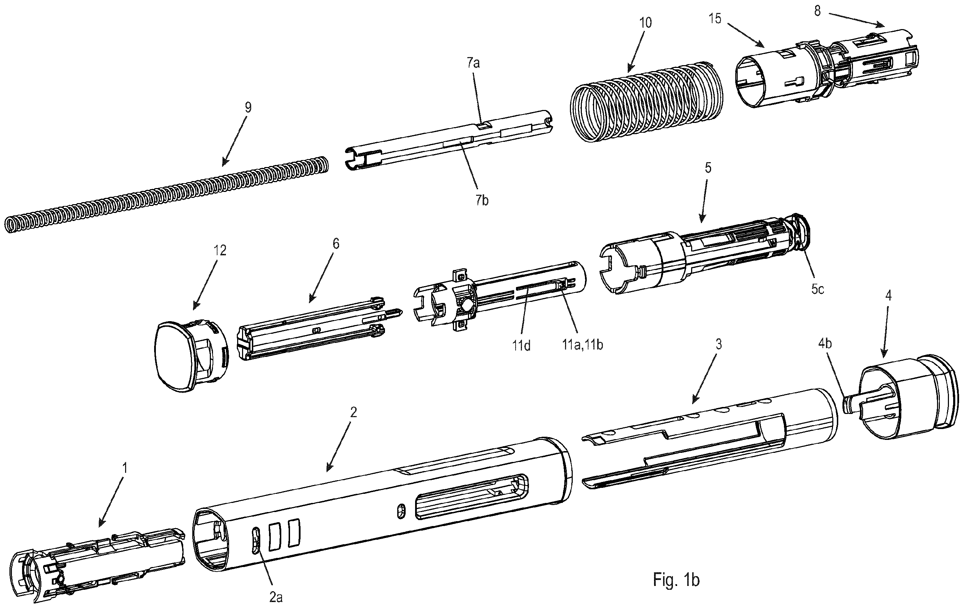

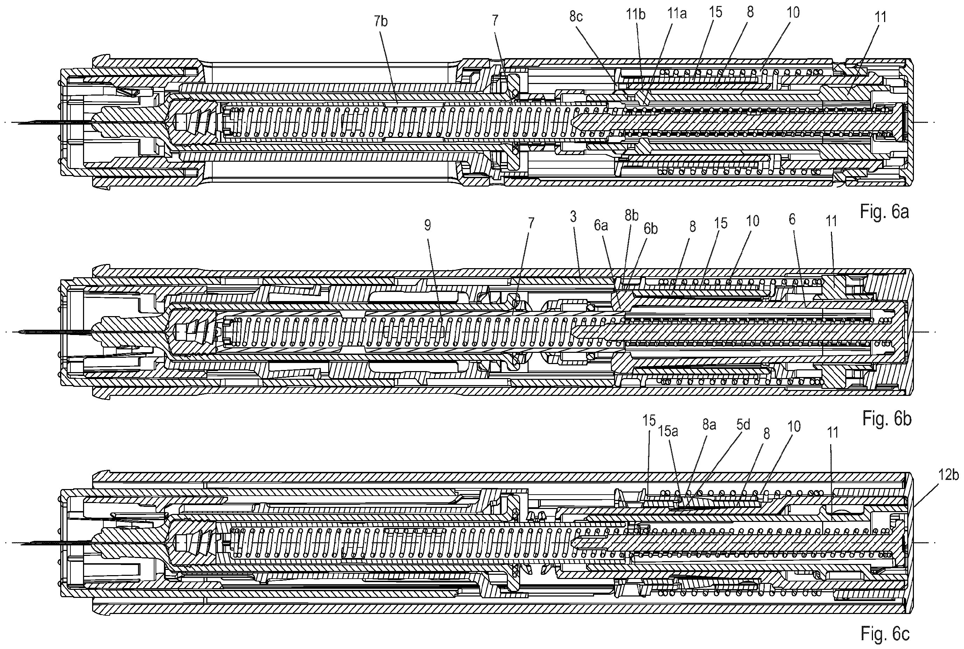

Bezugnehmend auf die Figuren 1-8 werden nachfolgend die strukturellen Merkmale und die Funktion des bevorzugten Autoinjektors beschrieben.

### WEGE ZUR AUSFÜHRUNG DER ERFINDUNG

Der Autoinjektor weist ein hülsenförmiges, längliches Gehäuse 2 mit einer Längsachse L auf, welches an seinem proximalen Ende eine Verschlusskappe 12 aufweist, die formschlüssig mit dem Gehäuse 2 dreh- und axialfest verbunden ist und das proximale Ende des Autoinjektors bildet. Die Verschlusskappe 12 ist mit dem Gehäuse 2 verschnappt. Hierfür weist die Verschlusskappe 12 ein Rastglied 12a auf, welches in eine Ausnehmung 2a am Gehäuse 2 eingerastet ist, vorzugsweise so, dass die Verschlusskappe 12 nicht oder nicht ohne weiteres von dem Gehäuse 2 lösbar ist.

Das Gehäuse 2 ist
i. aus einem unter Verwendung einer Mischung aus Recyclingöl und Rapsöl gebildetem Polypropylen, insbesondere von Purell^{™} HP671T renewable, erhältlich von der Firma LyondellBasell^{™}, oder
ii. aus einem unter Verwendung von Tallöl gebildetem Polymerblend enthaltend Polycarbonat und Acrylnitril-Butadien-Styrol, insbesondere von Cycoloy^{™} HCX1640 renewable erhältlich von der Firma Sabic^{™},
gebildet.

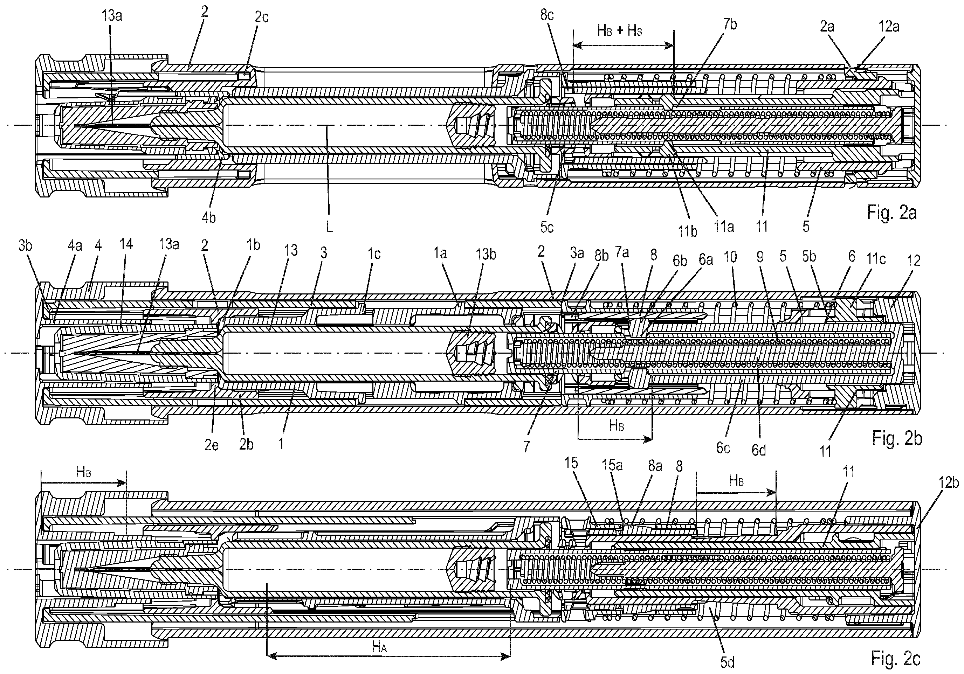

An dem distalen Ende des Autoinjektors ist in seinem Auslieferungszustand (Figuren 2a-2c) eine Abziehkappe 4 angeordnet, die vor der Verwendung des Autoinjektors abgezogen, oder abgedreht, und entfernt wird. Die Abziehkappe kann aus einem unter Verwendung einer Mischung aus Recyclingöl und Rapsöl gebildetem Polypropylen, insbesondere von Purell^{™} HP373P renewable, erhältlich von der Firma LyondellBasell^{™} oder aus einem unter Verwendung von Tallöl gebildeten Polycarbonat, insbesondere von Lexan^{™} HP2REU renewable, erhältlich insbesondere von der Firma Sabic^{™}, gebildet sein.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin das Produkt durch die hohle Injektionsnadel 13a aus dem Produktbehälter 13 ausschüttet. Der Spritzenkörper weist an seinem proximalen Ende einen sogenannten Fingerflansch auf, der radial nach außen über den Außenumfang des zylindrischen Spritzenkörpers ragt.

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 ist, wie am besten aus Figur 2a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen Spritzenkörperabschnitts, welcher den Kolben 13b führt, abstützt.

Um zu verhindern, dass der Produktbehälter 13 relativ zu dem Spritzenhalter 1 in die proximale Richtung verschiebbar ist, wird der Produktbehälter 13 an seinem proximalen Ende durch einen auf den Spritzenkörper wirkenden Halter in den Eingriff mit der Schulter 1b gedrückt. Der Halter wird von einem Haltefederabschnitt 5c eines Mechanikhalters 5 gebildet. Der Mechanikhalter 5 ist in Bezug auf das Gehäuse 2 entlang der Längsachse L insbesondere unverschiebbar und/oder drehfest angeordnet. Der hülsenförmige Mechanikhalter 5 kann mit dem Gehäuse 2 verschnappt sein. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

Der Produktbehälter 13 ist in Bezug auf das Gehäuse 2 so angeordnet, dass die Nadelspitze distal über das distale Ende des Gehäuses 2 übersteht. Im Ausgangs- oder Auslieferungszustand des Autoinjektors, d. h., wenn die Abziehkappe 4 an dem Autoinjektor angeordnet ist, wird die Nadel 13a von einer Nadelschutzkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Verschnappung wird in dem gezeigten Beispiel durch eine Schnappgeometrie 3b der Nadelschutzhülse 3 und einem Schnapphaken 4a der Abziehkappe 4 gebildet (Figur 2b). Diese Schnapphaken 4a sichern die Abziehkappe 4 weiter gegen eine proximale Bewegung relativ zum Gehäuse 2 indem sie auf dem Gehäuse 2 oder an einer distalen Stirnseite an dem Spritzenhalter 1 eine gehäusefeste Abstützung finden. Die Abziehkappe 4 weist ferner insbesondere an einem Schnapphaken 4a mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird. Alternativ kann der Schnapper 4b in eine Mantelfläche des rigid needle shield 14 oder in eine Mantelfläche des soft needle shield einhaken.

Der Autoinjektor weist eine Nadelschutzhülse 3 auf, die relativ zu dem Gehäuse 2 und entlang der Längsachse L um einen Betätigungshub H_{B} in die proximale Richtung in eine betätigte Position verschiebbar ist, um eine Produktausschüttung auszulösen. In der Ausgangsposition der Nadelschutzhülse 3, wie sie in den Figuren 2a-2c gezeigt wird, wobei die Abziehkappe 4 abgenommen ist, steht das distale Ende der Nadelschutzhülse 3 distal über die Nadelspitze der Nadel 13a über, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane Injektion erfolgen kann. Insbesondere kann das Gehäuse 2 einen Anschlag 2c bilden, an dem die Nadelschutzhülse 3 in der betätigten Position anliegt.

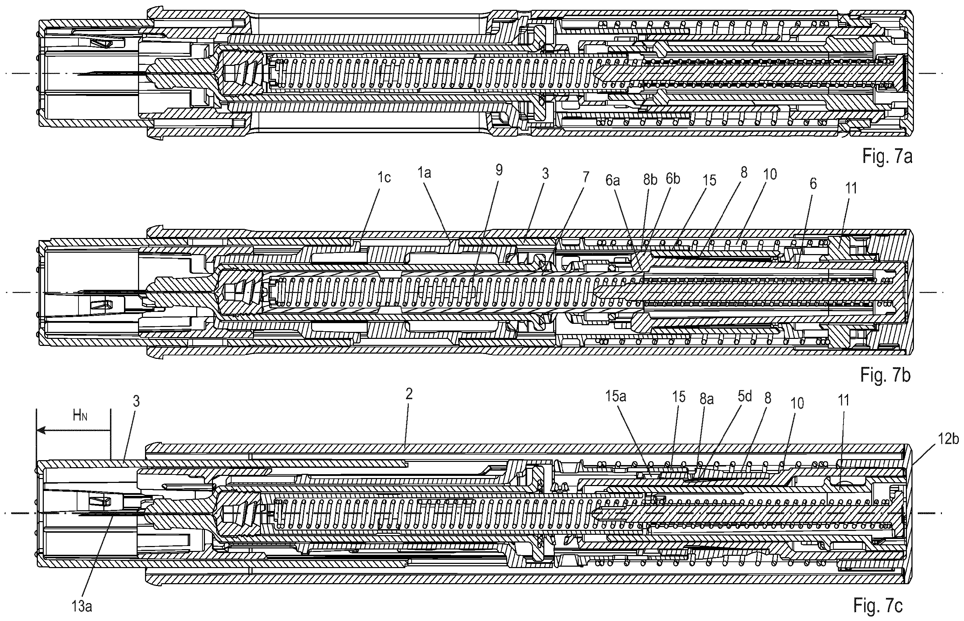

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figuren 7a-7c) verschoben werden. In der Nadelschutzposition steht das distale Ende der Nadelschutzhülse 3 distal über die Nadelspitze über, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spritzenhalter 1 weist eine Abragung 1a, die radial nach außen weist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 2a-2c) und/oder in der Nadelschutzposition der Nadelschutzhülse 3 (Figuren 7a-7c) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann ein Nocken 1c, der federnd an dem Spritzenhalter 1 angeordnet und von dem Spritzenhalter 1 gebildet ist, eingreifen. Der Nocken 1c ist so gestaltet, dass bei dem Versuch, die Nadelschutzhülse 3 aus der Ausgangsposition in die betätigte Position zu verschieben, der Nocken 1c die Verschiebung der Nadelschutzhülse 3 zunächst verhindert, wobei der Nocken 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 in die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 in die betätigte Position verschoben wird.

Das Gehäuse 2 weist einen zylinderförmigen Halteabschnitt oder Zylinderabschnitt 2b auf, der insbesondere das distale Ende des Spritzenhalters 1 insbesondere zylinderförmig umgibt, und daran anliegt, wodurch die mindestens eine Schulter 1b in dem Eingriff mit dem verjüngenden Bereich des Spritzenkörpers gehalten wird. Ferner weist das Gehäuse 2 im Bereich des Halteabschnitts 2b einen Translationsanschlag in der Gestalt einer Halteschulter 2e auf, die verhindert, dass der Spritzenhalter 1 relativ zu dem Gehäuse 2 in die distale Richtung verschiebbar ist, wenn der Spritzenhalter 1 an der Halteschulter 2e anliegt. Dies gilt auch vorteilhaft für die beschriebenen Varianten. Ferner kann der zylinderförmige Halteabschnitt 2b Rillen 2d aufweisen, welche in Verbindung mit Schienen 3c welche an der Innenseite der Nadelschutzhülse 3 angebracht sind eine Verdrehsicherung für die Nadelschutzhülse 3 bilden.

Der Autoinjektor weist ferner ein hülsenförmiges Vortriebsglied 7; 7' auf (Fig.8; Fig.9), welches an seinem distalen Ende nach innen und in Längsrichtung ragende Rippen 7c, 7c' bildet, an der sich eine erste Feder 9, die auch als Ausschüttfeder bezeichnet werden kann, abstützt. Die erste Feder 9 ist innerhalb des hülsenförmigen Vortriebsglieds 7; 7' angeordnet. Die Länge der Rippen 7c; 7c' ist so ausgestaltet, dass der Einbauraum für die erste Feder 9, die eine als Druckfeder wirkende Wendelfeder ist, verkleinert wird und so die Feder 9 im Ausgangs- oder Auslieferungszustand des Autoinjektors mit so viel Energie vorgespannt ist, dass sie das in dem Produktbehälter 13 enthaltene Produkt, insbesondere vollständig durch Verschieben des Vortriebsglieds 7; 7' um einen Ausschütthub H_{A} aus dem Produktbehälter 13 ausschütten kann. Zusätzlich bilden die Rippen 7c, 7c' eine Verstärkung des Bodens, des Vortriebsglieds 7; 7', so dass der distale Bereich des Vortriebsglieds 7; 7', bedingt durch die hohen Kräfte der Ausschüttfeder 9 nicht durchbricht. Die Länge der Rippen 7c; 7c' kann in verschiedenen Autoinjektoren unterschiedlich sein, wobei die entsprechende Länge der Rippen des Vortriebsglieds 7; 7' zur Einstellung der Federspannung der in den verschiedenen Autoinjektoren identischen ersten Feder dienen kann. Z.B. können länger ausgebildete Rippen eine höhere Federvorspannung erzeugen, zur Ausschüttung einer höherviskosen Flüssigkeit. Es können also zwei bis auf die Rippenlänge identische Autoinjektoren gebildet werden mit unterschiedlicher Federvorspannung. Es kann auch nur eine Rippe 7c; 7c' im entsprechenden Vortriebsglied 7; 7' vorgesehen sein. Im Auslieferungszustand der Vorrichtung besteht zwischen dem Kolben 13b und dem distalen Ende des Vortriebsglieds 7 ein Abstand, so dass das Vortriebsglied 7 erst während der Ausführung des Ausschütthubs H_{A} an den Kolben 13b anschlägt und diesen in die Ausschüttrichtung mitnimmt.

Die erste Feder 9 stützt sich mit ihrem proximalen Ende an einem Halteelement 6 ab, welches in diesem Beispiel zwei Arme 6c aufweist, wobei an jedem Arm 6c ein erstes Eingriffselement 6a und ein zweites Eingriffselement 6b angeordnet ist. Das Halteelement kann aus einem glasfaserverstärkten, unter Verwendung von aus fossilen Rohstoffen gebildeten Polyamid, insbesondere von Grilamid^{™} LV-65H FWA, erhältlich von der Firma EMS, gebildet sein. Das erste Eingriffselement 6a weist radial zu der Längsachse L hin, wobei das zweite Eingriffselement 6b radial von der Längsachse L weg weist. Das erste Eingriffselement 6a greift in eine erste Ausnehmung 7a, die von dem Vortriebselement 7 gebildet wird, ein, wodurch eine Bewegung des Vortriebsglieds 7 relativ zu dem Halteelement 6 in die distale Richtung oder in die Ausschüttrichtung verhindert wird. Hierdurch wird die erste Feder 9 in ihrem gespannten Zustand gehalten. Das Halteelement 6 weist einen Führungsstift 6d auf, der durch das proximale Ende der ersten Feder 9 in die Seele der Feder 9 eingefügt ist. Der Führungsstift 6d verhindert ein seitliches Ausknicken der ersten Feder 9 während und am Ende des Ausschütthubs H_{A} des Vortriebsglieds 7.

Der Autoinjektor weist ein Schaltmodul 8, 15 auf, welches eine Schalthülse 15 und eine von der Schalthülse 15 umgebene Sperrhülse 8 aufweist. Im Auslieferungszustand der Vorrichtung wird das erste Eingriffselement 6a von dem Innenumfang der Sperrhülse 8, der an dem zweiten Eingriffselement 6b anliegt, in dem Eingriff mit der ersten Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, innerhalb der die erste Feder 9 angeordnet ist und die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an einem Signalglied 11, insbesondere an einer Abragung 11c, die axial verschiebbar und verdrehfest in das Gehäuse 2 eingreift und die durch eine schlitzförmige Nut 5b des Mechanikhalters 5 hindurchgreift, ab. Die zweite Feder 10 umgibt somit auch den Mechanikhalter 4 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub H_{B} mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Das insbesondere hülsenförmige Signalglied 11 ist im Auslieferungszustand oder vor der Auslösung der Produktausschüttung in einem axialfesten Eingriff mit dem Vortriebsglied 7. Das Signalglied 11 weist ein erstes Eingriffsglied 11a, welches in eine Ausnehmung 7b des Vortriebsglieds 7 eingreift, und ein zweites Eingriffsglied 11b auf. Das erste Eingriffsglied 11a und das zweite Eingriffsglied 11b sind an dem Ende eines Arms 11d federnd angeordnet. Das Signalglied 11 weist zwei solcher Arme 11d mit einem ersten Eingriffsglied 11a und einem zweiten Eingriffsglied 11b auf. Das erste Eingriffsglied 11a weist radial zu der Längsachse L hin, wobei das zweite Eingriffsglied 11b radial von der Längsachse L weg weist. Im Auslieferungszustand wird das erste Eingriffsglied 11a von dem Innenumfang der Sperrhülse 8 in dem axialfesten Eingriff mit dem Vortriebsglied 7 gehalten. In alternativen Ausführungen kann die Ausnehmung 7b derart ausgebildet sein, insbesondere eine in Längsrichtung erstreckende Ausnehmung 7b sein, dass während eines ersten Teilhubs des Ausschütthubs eine axiale Relativbewegung zwischen dem Vortriebsglied 7 und dem Signalglied 11 stattfindet und während eines zweiten Teilhubs des Ausschütthubs das erste Eingriffsglied 11a in dem axialfesten Eingriff zumindest in distaler Richtung mit dem Vortriebsglied 7 gehalten wird. Das zweite Eingriffsglied 11b liegt an dem Innenumfang der Schalthülse 8 an. Die Verschlusskappe 12 weist einen Signalanschlag 12b auf, an den das Signalglied 11 zur Erzeugung eines Signals anschlagen kann und vorzugsweise an dem das Signalglied 11 im Auslieferungszustand der Vorrichtung anliegt.

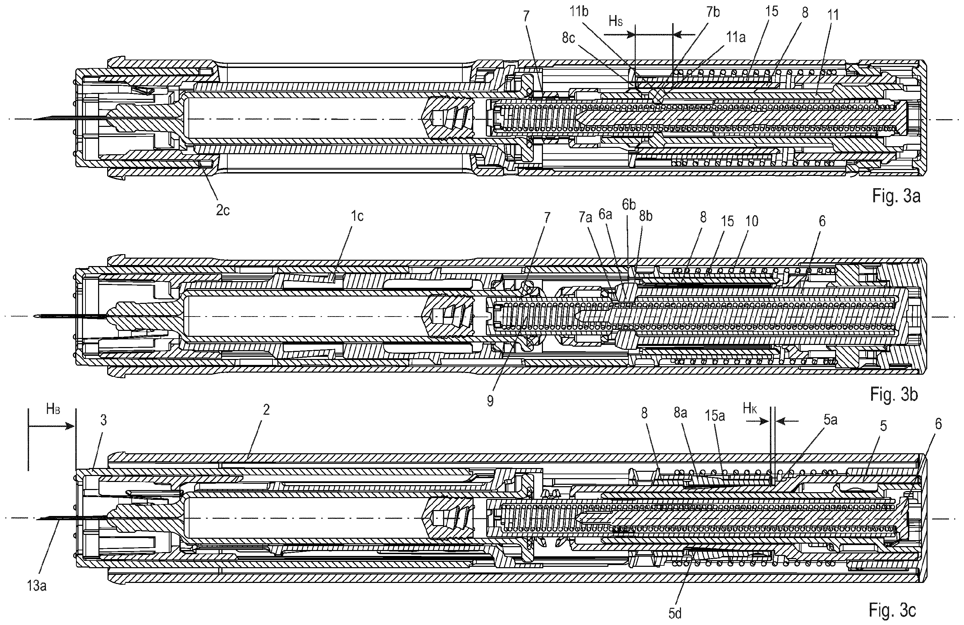

Zur Verabreichung des Produkts aus dem Produktbehälter 13 wird die Abziehkappe 4 von dem Autoinjektor zusammen mit dem rigid needle shield 14 entfernt. Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub H_{B} in die proximale Richtung relativ zu dem Gehäuse 2 in die betätigte Position bewegt. Hierdurch wird die zweite Feder 10 gespannt, wobei die Schalthülse 15 von der Nadelschutzhülse 3 um den Betätigungshub H_{B} mitgenommen wird. Die Sperrhülse 8 weist eine erste Ausnehmung 8b auf, die durch Verschieben der Sperrhülse 8 um den Betätigungshub H_{B} entlang der Längsachse L auf die Position des zweiten Eingriffselements 6b gebracht wird, wie in den Figuren 3a-3c dargestellt. Hierdurch wird das erste Eingriffselement 6a aus dem Eingriff mit dem Vortriebsglied 7 mit einer Bewegung quer zu und von der Längsachse L weg bewegt, wobei gleichzeitig das zweite Eingriffselement 6b in den Eingriff mit der Sperrhülse 8, insbesondere deren erster Ausnehmung 8b bewegt wird. Hierdurch wird das Vortriebsglied 7 für die Bewegung um den Ausschütthub H_{A} in die Ausschüttrichtung freigegeben.

Da die axialfeste Kopplung zwischen dem Vortriebsglied 7 und dem Halteelement 6 nun aufgehoben ist, kann das Halteelement 6, welches zumindest ein Stück weit relativ zu dem Gehäuse 2 und entlang der Längsachse L bewegbar ist, von der ersten Feder 9 in die proximale Richtung bewegt werden, wobei das Halteelement 6 über den Eingriff des zweiten Eingriffselements 6b in die Ausnehmung 8b die Sperrhülse 8 um einen Startsignalhub H_{K} (Figur 3c) mitnimmt, wodurch die Sperrhülse 8 an einem Startsignalanschlag 5a, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 8 um den Betätigungshub H_{B} wird das Verriegelungsglied 8a für eine Bewegung quer und zu der Längsachse L hin freigegeben, da der Mechanikhalter 5 eine Vertiefung 5d aufweist, welche eine solche Bewegung des Verriegelungsglieds 8a zulässt, wenn die Sperrhülse 8 um den Betätigungshub H_{B} verschoben wurde oder wenn die Nadelschutzhülse 3 in ihrer betätigten Position ist.

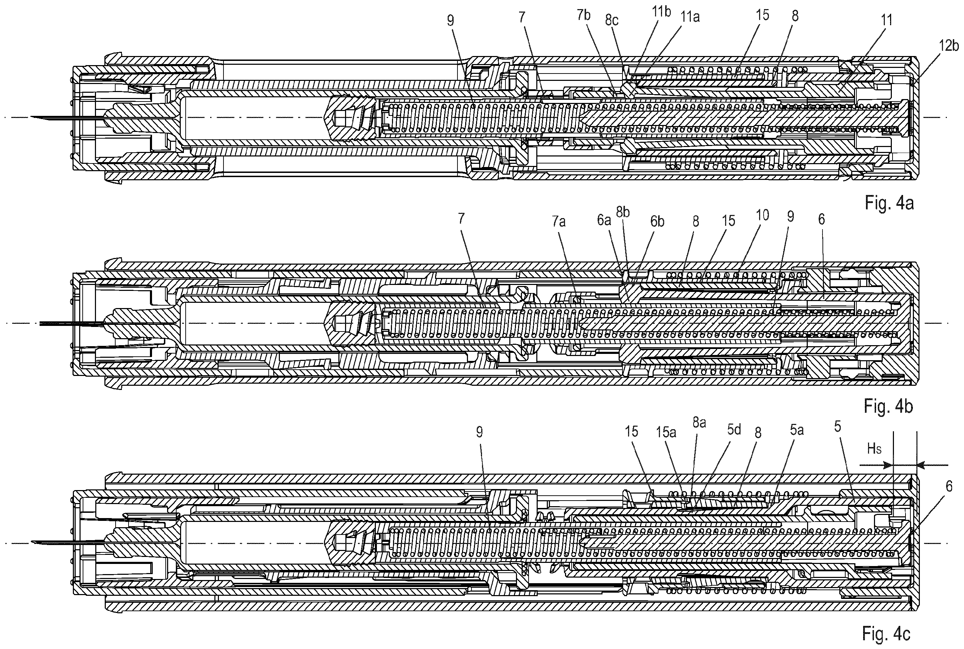

Da das Signalglied 11 noch axialfest mit dem Vortriebsglied 7 verbunden ist, wird es um einen ersten Teilhub Hs des Ausschütthubs H_{A} in die Ausschüttrichtung mitgenommen, wobei das Signalglied 11 in etwa um den ersten Teilhub Hs von dem Signalanschlag 12b weg bewegt wird, wie am besten aus Figur 4c erkennbar ist. Am Ende des ersten Teilhubs Hs, während dem das erste und zweite Eingriffsglied 11a, 11b relativ zu der Sperrhülse 8 bewegt werden, wird das erste Eingriffsglied 11a aus dem Eingriff mit dem Vortriebsglied 7 gedrückt, wobei gleichzeitig das zweite Eingriffsglied 11b in die zweite Ausnehmung 8c der Sperrhülse 8 mit einer Bewegung quer zu der Längsachse L und radial von der Längsachse L weg bewegt wird. Hierdurch wird das Signalglied 11 daran gehindert, sich in die proximale Richtung relativ zu dem Gehäuse 2 oder der Sperrhülse 8 zu bewegen. Das zweite Eingriffsglied 11b wird von dem Außenumfang des Vortriebsglieds 7 in den Eingriff mit der Ausnehmung 8c gehalten (Figur 4a), wenn das Vortriebsglied 7 um seinen zweiten Teilhub des Ausschütthubs H_{A} bewegt wird. Die Außenumfangsfläche des Vortriebsglieds 7 hält das zweite Eingriffselement 6b in dem Eingriff mit der ersten Ausnehmung 8b der Sperrhülse 8, wie am besten aus Figur 4b erkennbar ist. Am Ende des Ausschütthubs H_{A} gibt das Vortriebsglied 7 das zweite Eingriffsglied 11b aus dem Eingriff mit der Sperrhülse 8 frei, wodurch das zweite Eingriffsglied 11b aus dem Eingriff mit der Ausnehmung 8c bewegt wird, insbesondere zu der Längsachse L hin, so dass die zweite Feder 10 das Signalglied 11 entgegen die Ausschüttrichtung, d. h. in die proximale Richtung beschleunigt, so dass beim Auftreffen des Signalglieds 11 auf dem Signalanschlag 12b ein akustisches und/oder taktiles Signal erzeugt wird.

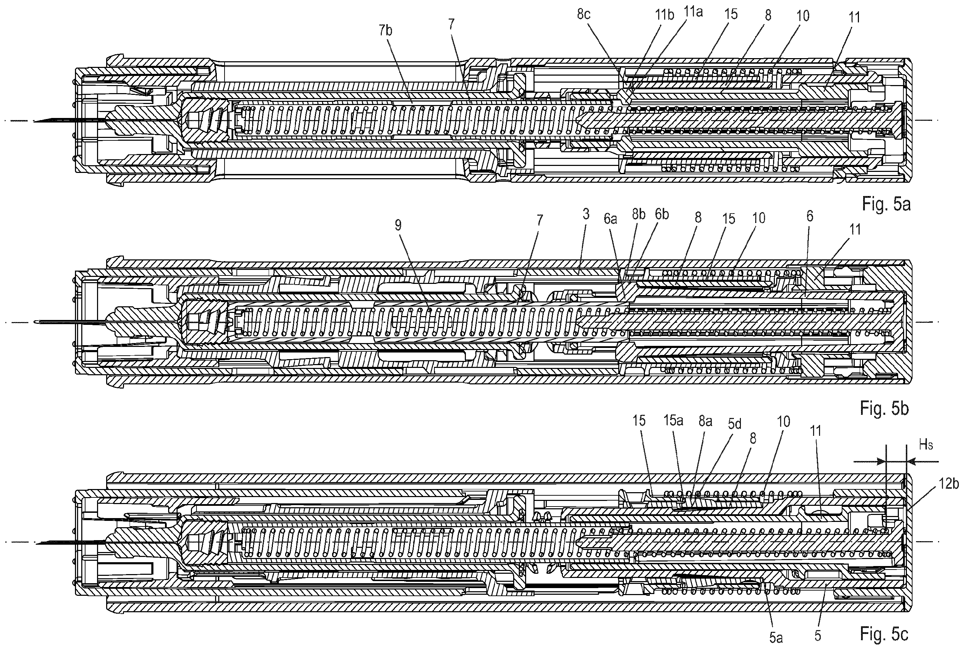

Wie am besten aus Figur 5b erkennbar ist, bleibt der Eingriff des zweiten Eingriffselements 6b in die erste Ausnehmung 8b bestehen, wodurch eine Bewegung der Sperrhülse 8 in die distale Richtung relativ zu dem Gehäuse 2 verhindert wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub H_{N} bewegen, wobei das Verriegelungsglied 8a aus dem Eingriff mit der Ausnehmung 15a gedrückt wird, wobei sich die Schalthülse 15 relativ zu der Sperrhülse 8 in die distale Richtung bewegt. Wenn die Nadelschutzhülse 3 in ihrer Nadelschutzposition ist, verschnappt das Verriegelungsglied 8a mit der Schalthülse 15, wobei das Verriegelungsglied 8a ein Zurückschieben der Nadelschutzhülse 3 in ihre betätigte Position verhindert. Bei dem Versuch, die Nadelschutzhülse 3 aus der Nadelschutzposition in die betätigte Position zurückzuschieben, stößt das Schaltglied 15 an dem Verriegelungsglied 8a an, welches die Bewegung der Nadelschutzhülse 3 in die betätigte Position verhindert. Die Sperrhülse 8 stützt sich hierzu axial an dem Startsignalanschlag 5a des Mechanikhalters 5 ab.

**BEZUGSZEICHENLISTE**

| | | | |
|---|---|---|---|
| 1 | Spritzenhalter | 8 | Sperrhülse |
| 1a | Abragung/ Halteglied | 8a | Verriegelungsglied |
| 1b | Schulter/ Eingriffsglied | 8b | erste Ausnehmung |
| 1c | Nocken | 8c | zweite Ausnehmung |
| 2 | Gehäuse | | |
| 2a | Ausnehmung | 9 | erste Feder/Ausschüttfeder |
| 2b | Zylinderabschnitt/Halteabschnitt | 10 | zweite Feder/Nadelschutzfeder |
| 2c | Betätigungsanschlag | | |
| 2e | Halteschulter | 11 | Signalglied |
| 2d | Rillen | 11a | erstes Eingriffsglied |
| | | 11b | zweites Eingriffsglied |
| 3 | Nadelschutzhülse | 11c | Abragung |
| 3a | proximales Ende | 11d | Arm |
| 3b | Schnappgeometrie | | |
| 3c | Schienen | 12 | Verschlusskappe |
| | | 12a | Rastglied |
| 4 | Abziehkappe | 12b | Signalanschlag |
| 4a | Schnapphaken | | |
| 4b | Schnapper | 13 | Produktbehälter/Spritze |
| | | 13a | Nadel |
| 5 | Mechanikhalter | 13b | Kolben |
| 5a | Startsignalanschlag | | |
| 5b | Nut | 14 | rigid needle shield/ Nadelschutzkappe |
| 5c | Haltefederabschnitt | | |
| 5d | Vertiefung | 15 | Schalthülse |
| | | 15a | Ausnehmung |
| 6 | Halteelement | | |
| 6a | erstes Eingriffselement | H_{A} | Ausschütthub |
| 6b | zweites Eingriffselement | H_{B} | Betätigungshub |
| 6c | Arm | Hs | Signalhub/erster Teilhub |
| 6d | Führungsstift | H_{K} | Startsignalhub |
| | | H_{N} | Nadelschutzhub |
| 7; 7' | Vortriebsglied | H_{M} | Montagehub |
| 7a | erste Ausnehmung | | |
| 7b | zweite Ausnehmung | L | Längsachse |
| 7c; 7c' | Rippen | | |

## Patentansprüche

1. Autoinjektor zur Ausschüttung eines flüssigen Produkts, insbesondere Medikaments, umfassend:
a. ein Gehäuse (2) und einen in dem Gehäuse (2) angeordneten Produktbehälter (13), insbesondere Spritze, der einen verschiebbaren Kolben (13b) aufweist, wobei der Kolben (13b) in eine Ausschüttrichtung verschiebbar ist, um eine Produktausschüttung des in dem Produktbehälter (13) enthaltenen Produkts zu bewirken,
b. ein Vortriebsglied (7; 7'), das während der Produktausschüttung auf den Kolben (13b) wirkt, und eine erste Feder (9), die auf das Vortriebsglied (7; 7') wirkt,
c. eine Nadelschutzhülse (3), welche für die Auslösung der Produktausschüttung aus einer Ausgangsposition relativ zu dem Gehäuse (2) und entlang einer Längsachse (L) des Autoinjektors in eine proximale Richtung verschiebbar ist, wodurch eine zweite Feder (10) gespannt und insbesondere die Produktausschüttung ausgelöst wird, wobei die Nadelschutzhülse (3) auf die zweite Feder (10) wirkt,
**dadurch gekennzeichnet, dass**
d. das Gehäuse (2)
i. aus einem unter Verwendung einer Mischung aus Recyclingöl und Rapsöl gebildeten Polypropylen, insbesondere von Purell^{™} HP671T renewable, erhältlich insbesondere von der Firma LyondellBasell^{™}, oder
ii. aus einem unter Verwendung von Tallöl gebildeten Polymerblend enthaltend Polycarbonat und Acrylnitril-Butadien-Styrol, insbesondere von Cycoloy^{™} HCX1640 renewable, erhältlich insbesondere von der Firma Sabic^{™},
gebildet ist.

2. Autoinjektor nach Anspruch 1, **dadurch gekennzeichnet, dass** an einem distalen Ende des Autoinjektors eine Abziehkappe (4) angeordnet ist, welche vor der Verwendung des Autoinjektors abgezogen, abgedreht oder auf andere Weise entfernt werden kann, und welche
a. aus einem unter Verwendung einer Mischung aus Recyclingöl und Rapsöl gebildeten Polypropylen, insbesondere von Purell^{™} HP373P renewable, erhältlich insbesondere von der Firma LyondellBasell^{™}, oder
b. aus einem unter Verwendung von Tallöl gebildeten Polycarbonat, insbesondere von Lexan^{™} HP2REU renewable, erhältlich insbesondere von der Firma Sabic^{™},
gebildet ist.

3. Autoinjektor nach Anspruch 1 oder 2, **dadurch gekennzeichnet, dass** die Nadelschutzhülse (3) aus einem unter Verwendung von Biogas gebildeten Polyoxymethylen-Copolymer, insbesondere Hostaform^{™} MT12R01 renewable, erhältlich insbesondere von der Firma Celanese^{™}, gebildet ist.

4. Autoinjektor nach einem der vorhergehenden Ansprüche, **dadurch gekennzeichnet, dass** der Autoinjektor einen Spritzenhalter (1) umfasst, der den Produktbehälter (13) axialfest hält und axialfest mit dem Gehäuse (2) verbunden ist, und welcher aus einer unter Verwendung von Tallöl gebildeten Poycarbonat / Polyester-Legierung, insbesondere Cylex^{™} HX7509HP renewable, erhältlich insbesondere von der Firma Sabic^{™}, gebildet ist.

5. Autoinjektor nach einem der vorhergehenden Ansprüche, **dadurch gekennzeichnet, dass** der Autoinjektor an einem proximalen Ende des Gehäuses (2) eine Verschlusskappe (12) aufweist, welche insbesondere form-, kraft- oder stoffschlüssig mit dem Gehäuse verbunden ist, und welche aus einem unter Verwendung von Tallöl gebildeten Polymerblend enthaltend Polycarbonat und Acrylnitril-Butadien-Styrol, insbesondere von Cycoloy^{™} HCX1640 renewable, erhältlich insbesondere von der Firma Sabic^{™}, gebildet ist.

6. Autoinjektor nach einem der vorhergehenden Ansprüche, **dadurch gekennzeichnet, dass** das Vortriebsglied (7; 7') aus einem unter Verwendung von Biogas gebildeten Polyoxymethylen-Copolymer, insbesondere Hostaform^{™} MT12R01 renewable, erhältlich insbesondere von der Firma Celanese^{™}, gebildet ist.

7. Autoinjektor nach einem der vorhergehenden Ansprüche, **dadurch gekennzeichnet, dass** der Autoinjektor ein Halteelement (6) aufweist, welches eine Bewegung des Vortriebsglieds (7; 7') relativ zu dem Halteelement (6) und/oder dem Gehäuse (2) in eine Ausschüttrichtung vor einem Auslösen der Produktausschüttung verhindert, wobei das Halteelement aus einem glasfaserverstärkten, unter Verwendung von aus fossilen Rohstoffen gebildeten Polyamid, insbesondere von Grilamid^{™} LV-65H FWA, erhältlich insbesondere von der Firma EMS, gebildet ist.

8. Autoinjektor nach einem der vorangehenden Ansprüche, **dadurch gekennzeichnet, dass** der Autoinjektor ein Schaltmodul umfasst, welches insbesondere
a. aus einem unter Verwendung von Biogas gebildeten Polyoxymethylen-Copolymer, insbesondere Hostaform^{™} MT12R01 renewable, erhältlich insbesondere von der Firma Celanese^{™}, oder
b. aus einem unter Verwendung von Biogas gebildeten Polyoxymethylen-Homopolymer, insbesondere Delrin^{™} SC698 renewable, erhältlich insbesondere von der Firma DuPont^{™},
gebildet ist, und insbesondere eine Sperrhülse (8) und/oder eine Schalthülse (15) umfasst.

9. Autoinjektor nach einem der vorhergehenden Ansprüche, **dadurch gekennzeichnet, dass** der Autoinjektor einen Signalglied (11) umfasst, welches aus einem unter Verwendung von Therephthalat aus PET-Flaschen-Recycling gebildeten, glasfaserverstärkten Polybutylenterephthalat (PBT), insbesondere Elcrin^{™} WF006JiQ, erhältlich insbesondere von der Firma Sabic^{™}, gebildet ist.

10. Ein Set von Autoinjektoren zur Ausschüttung eines flüssigen Produkts, umfassend:
a. einen ersten Autoinjektor gemäss einem der Ansprüche 1 bis 9,
b. einen zweiten Autoinjektor gemäss dem Oberbegriff von Anspruch 1, bei welchem das Gehäuse (2) aus, unter ausschliesslicher Verwendung von fossilen Rohstoffen gebildetem, Kunststoff, insbesondere aus einem Polymerblend enthaltend Polycarbonat und Acrylnitril-Butadien-Styrol Polyamid, gebildet ist.

11. Ein Set von Autoinjektoren gemäss Anspruch 10, bei welchem Gehäuse (2), Produktbehälterhalter bzw. Spritzenhalter (1), Nadelschutzhülse (3), Mechanikhalter (5), Vortriebsglied (7), Signalglied (11), Schaltmodul, Schalthülse (15), Sperrhülse (8), Verschlusskappe (12), Halteelement (6), und/oder Abziehkappe (4) des ersten und zweiten Autoinjektors geometrisch, strukturell und/oder funktional paarweise zueinander identisch sind.

12. Ein Set von Autoinjektoren gemäss Anspruch 10 oder 11, bei welchem sämtliche funktional einander entsprechenden mechanischen Bestandteile des ersten und zweiten Autoinjektors geometrisch und/oder strukturell paarweise zueinander identisch sind.

13. Ein Set von Autoinjektoren gemäss einem der Ansprüche 10 bis 12, bei welchem sämtliche mittels Giessverfahren, insbesondere mittels Spritz- und/oder Druckguss, hergestellten einander entsprechenden Kunststoffteile - insbesondere Gehäuse (2), Produktbehälterhalter bzw. Spritzenhalter (1), Nadelschutzhülse (3), Mechanikhalter (5), Vortriebsglied (7), Signalglied (11), Schaltmodul, Schalthülse (15), Sperrhülse (8), Verschlusskappe (12), Halteelement (6), und/oder Abziehkappe (4) - jeweils unter Verwendung identischer, insbesondere jeweils eines einzigen, Spritzgusswerkzeugs hergestellt sind.
